# EUROPEAN PATENT APPLICATION

(11) **EP 4 356 928 A1**
(43) Date of publication of application: **24.04.2024**
(21) Application number: 22202808.6
(22) Date of filing: 20.10.2022
(51) Int. Cl.: A61K 47/54, A61P 31/12

(54) **NUCLEOSIDE DIPHOSPHATE OR DIPHOSPHONATE PRODRUGS, OR NUCLEOSIDE ANALOGUE DIPHOSPHATE OR DIPHOSPHONATE PRODRUGS**

(71) Applicant: Universität Hamburg, 20148 Hamburg (DE)
(72) Inventor: Meier, Chris, 21635 Jork (DE); Jia, Xiao, 20146 Hamburg (DE)
(74) Representative: Stüven, Ralf

(57) **Abstract**

The invention provides a nucleoside diphosphate or nucleoside diphosphonate prodrug or a nucleoside analogue diphosphate or nucleoside analogue diphosphonate prodrug, or a pharmaceutically acceptable salt thereof, having two covalently bound lipophilic moieties A and B at the terminal phosphate group, phosphonate group or analogous phosphorus-containing group, wherein the lipophilic moieties A and B are both non-bioreversible.

## Description

The invention relates to nucleoside diphosph(on)ate prodrugs or nucleoside analogue diphosph(on)ate prodrugs.

Nucleoside analogues, which can be incorporated into DNA, are not only used in the treatment of, for example, viral infectious diseases such as herpes, hepatitis, the immunodeficiency disease AIDS (Acquired Immunodeficiency Syndrome) or even Covid-19, but also in cancer. After incorporation, the nucleoside analogues act, for example, as chain terminators (chain-terminating nucleoside analogues, CTNA) because the nucleoside analogues lack a 3' OH group, such that there is no further elongation possible in the 3'-direction (Balzarini, P. Herdewijn, E. De Clercq. Differential Patterns of intracellular Metabolism of 2',3'-Didehydro-2',3'-dideoxythymidine and 3'-Azido-2',3'-dideoxythymidine, two potent Anti-human Immunodeficiency Virus Compounds, J. Biol. Chem. 1989, 264, 6127-6133). For this purpose, however, the nucleoside analogues must be present as triphosphates (NTP), so that they can be incorporated into the DNA or RNA strand for chain extension.

In the form of their mono-, di- or triphosphates (NMP, NDP, NTP), nucleoside analogues cannot penetrate the cell membrane under physiological conditions due to their charge. It is also not possible to pass the blood brain barrier (BBB) to treat diseases affecting the brain. Therefore, nucleoside analogues must either be intracellularly converted into their triphosphates by more or less specific kinases in several stages, or phosphorylated nucleoside analogues must be designed or modified so as to be able to pass through the cell membrane or BBB.

For this purpose, the lipophilicity of the drugs has usually to be increased (e.g., RJ Sawchuk, Z. Yang. Investigation of distribution, transport and uptake of anti-HIV drugs to the central nervous system, Advanced Drug Delivery Reviews. 1999, 39, 5-31). One possibility of increasing the lipophilicity of known drugs is the use of prodrug systems. Prodrugs are precursors of an active agent, releasing the active agent at the desired site of action after biotransformation, e.g., detachment of masking groups. In addition to increased lipophilicity, further preconditions for prodrugs are sufficient stability in extracellular medium and the release of non-toxic masks.

Enzymatically activatable prodrug systems for nucleoside monophosphates (NMP) have already been described (A. Pompon, I. Lefebvre, J.-L. Imbach, S. Khan, D. Farquhar. Decomposition Pathways of the Mono-(Pivaloyloxymethyl) and Bis-(Pivaloyloxymethyl) Esters of Azidothymidine-5'-Monophosphate in Cell Extract and in Tissue-Culture Medium - An Application of the Online ISRP-Cleaning HPLC Technique, Antiviral Chem. Chemother. 1994, 5, 91-98; I. Lefebvre, C. Perigaud, A. Pompon, A.-M. Aubertin, J.-L. Girardet, A. Kim, G. Gosselin, J.-L. Imbach. Mononucleoside Phosphotriester Derivates with S Acyl-2-thioethyl Bioreversible Phosphate-Protecting Groups: Intracellular Delivery of 3'-Azido-2',3'-dideoxythymidine-5'-monophosphate, J. Med. Chem. 1995, 38, 3941-3950; W. Thomson, D. Nicholls, W. J. Irwin, J. S. Al-Mushadani, S. Freeman, A. Karpas, J.Petrik, N. Mahmood, A. J. Hay. Synthesis, Bioactivation and Anti-HIV Activity of the Bis(4-acyloxybenzyl) and Mono(4-acyloxybenzyl) Esters of the 5'-Monophosphate of AZT, J. Chem. Soc., Perkin Trans, 1993, 1, 1239-1245; A. Routledge, I. Walker, S. Freeman, A. Hay, N. Mahmood. Synthesis, Bioactivation and Anti-HIV Activity of 4-Acyloxybenzyl Bis(Nucleosid-5-yl) Phosphates, Nucl. Nucl. 1995, 14, 1545-1558; C. Meier. CycloSal Phosphates as Chemical trojan Horses for the intracellular Nucleotide and Glycosylmonophosphate Delivery - Chemistry meets Biology, Eur. J. Org. Chem. 2006, 1081-1102). An example of a known and successfully applied nucleoside monophosphate prodrug is Sofosbuvir, which is used in the treatment of chronic Hepatitis C. NMP prodrugs are, however, are considered disadvantageous because the subsequent phosphorylation to diphosphates and triphosphates in the cell can still be inhibited or completely suppressed. For example, in the case of the nucleoside analogue azidothymidine (AZT), a known anti-HIV drug, the phosphorylation to the AZTDP (DP = diphosphate) is inhibited. In addition, numerous side effects are attributed to the corresponding monophosphate AZTMP (MP = monophosphate).

Consequently, there have been several attempts to design suitably masked nucleoside triphosphates (NTP) or nucleoside diphosphates (NDP) and analogues thereof in order to allow the introduction of prodrugs releasing the corresponding NDP or NTP in the cell.

WO 2009/129798 A2 relates to compounds that can be used as prodrugs, medicaments, pharmaceutical compositions, and pharmaceutical forms of administration produced from said compounds, and a method for producing the compounds according to the invention. The invention proposes bioreversible masked diphosphates and triphosphates, in particular nucleoside diphosphates (NDP) and nucleoside triphosphates (NTP), and corresponding analogues suitable for treating viral diseases such as AIDS, for example.

WO 2016/026493 A1 relates to compounds which can be used as prodrugs, in particular nucleoside diphosphate and triphosphate prodrugs, and to a method for producing said compounds. The document describes nucleotide or nucleotide analogue prodrugs that are bioreversibly and asymmetrically masked only at the terminal phosphate, i.e., at the β- or γ-phosphate, whereas the masking of the internal phosphate(s) is omitted.

The delivery system described in WO 2009/129798 A2 and WO 2016/026493 A1 is also known as DiPPro or TriPPPro approach, and the respective prodrugs as DiPPro or TriPPPro compounds, depending on the masking of nucleoside di- or triphosphates (see, for example, Jessen HJ, Schulz T, Balzarini J, Meier C. Bioreversible protection of nucleoside diphosphates, Angew. Chem. Int. Ed. 2008;47(45):8719-22, doi: 10.1002/anie.200803100; T. Gollnest, T. Dinis de Oliveira, A. Rath, I. Hauber, D. Schols, J. Balzarini, C. Meier. Membrane-permeable Triphosphate Prodrugs of Nucleoside Analogues, Angew. Chem. Int. Ed. 2016, 55, 5255, doi: 10.1002/anie.201511808; Meier C. Nucleoside diphosphate and triphosphate prodrugs - An unsolvable task? Antiviral Chem. Chemother. 2017 Dec;25(3):69-82. doi: 10.1177/2040206617738656; Jia X, Schols D, Meier C. Lipophilic Triphosphate Prodrugs of Various Nucleoside Analogues, J. Med. Chem. 2020 Jul 9;63(13):6991-7007, doi: 10.1021/acs.jmedchem.0c00358; Jia X, Schols D, Meier C. Anti-HIV-Active Nucleoside Triphosphate Prodrugs, J. Med. Chem. 2020 Jun 11;63(11):6003-6027, doi: 10.1021/acs.jmedchem.0c00271; Zhao C, Jia X, Schols D, Balzarini J, Meier C. γ-Non-Symmetrically Dimasked TriPPPro Prodrugs as Potential Antiviral Agents against HIV, ChemMedChem. 2021 Feb 4;16(3):499-512. doi: 10.1002/cmdc.202000712; Jia X, Ganter B, Meier C. Improving properties of the nucleobase analogs T-705/T-1105 as potential antiviral. Annu. Rep. Med. Chem. 2021, 57:1-47, doi: 10.1016/bs.armc.2021.08.002). Acyloxybenzyl groups are used in this concept to mask the terminal phosphate group. In the past few years, this general concept has been modified in that nucleoside triphosphate and nucleoside triphosphate analogue prodrugs have been developed having, at the γ-phosphate, an intracellular stable first mask and an intracellular labile (biocleavable) second mask B, such that a nucleoside triphosphate or analogue thereof mono-modified with the intracellularly stable moiety is released (WO 2018/100137 A1). More recently, acyloxybenzyl and alkoxycarbonyloxybenzyl prodrugs of γ-alkyl-modified modified stavudine (d4T) triphosphate and γ-dialkyl-modified stavudine (d4T) triphosph(on)ate prodrugs have been described, having one or two non-bioreversible γ-alkyl masking groups (C. Zhao, S. Weber, D. Schols, J. Balzarini, C. Meier. Prodrugs of γ-Alkyl-Modified Nucleoside Triphosphates: Improved Inhibition of HIV Reverse Transcriptase, Angew. Chem. Int. Ed. 2020, 59, 22063-22071; Jia X, Weber S, Schols D, Meier C. J. Med. Chem. 2020, 63, 20, 11990-12007; Jia X. Membrane-Permeable Nucleoside Triphosphate Prodrugs of Anti-HIV Active Nucleoside Analogues: γ-(Phosphate or Phosphonate)-Modified Nucleotide Analogues, doctoral dissertation, 2020, University of Hamburg, Hamburg, Germany, https://d-nb.info/1245000837).

In view of the fact that nucleotide or nucleotide analogue prodrugs have a broad range of possible applications, and that it is of interest to be able to choose from a variety of different nucleotide or nucleotide analogue prodrugs, for example, in order to avoid or minimize possible adverse effects or resistance, there is still a need for novel nucleotide or nucleotide analogue prodrugs. It is therefore an object of the present invention to provide new and improved nucleotide or nucleotide analogue prodrugs and/or acceptable salts thereof.

The problem is solved according to the invention by a symmetric or non-symmetric chemical modification of nucleoside diphosphate and/or nucleoside diphosphonate compounds, or analogues thereof, at the terminal β-phosph(on)ate group, or the respective analogous group containing a β-phosphorus atom, resulting in prodrugs being non-bioreversible but chemically cleavable under release of the corresponding unmasked drug comprising a monophosph(on)ate group or analogous group containing an α-phosphorus atom.

Advantages of the compounds according to the invention are, for example, that the compounds provide high lipophilicity for entry into cells and have a readily available syntheses route with high yields. In this context, it is of advantage that only one phosphate (or analogous P-containing) group is left unmasked, leading to higher lipophilicity compared to triphosphates, for example. Further, it has surprisingly been found that the compounds of the invention have a very high antiviral activity, even in the form of the prodrug itself, i.e., comprising both or least one of the covalently bound masks. The prodrugs of the invention are substrate for e.g. HIV reverse transcriptase, other than corresponding triphosphate prodrugs. Due to the masking by the non-bioreversible moieties the corresponding nucleoside monophosphates are formed very slowly in the cell.

In a first aspect the present invention provides a nucleoside diphosphate or nucleoside diphosphonate prodrug or a nucleoside analogue diphosphate or nucleoside analogue diphosphonate prodrug, or a pharmaceutically acceptable salt thereof, having two covalently bound lipophilic moieties A and B at the terminal phosphate group, phosphonate group or analogous phosphorus-containing group, wherein the lipophilic moieties A and B are both non-bioreversible. At least one of the moieties A or B neutralizes and/or covers any negative electric charge at the single-bonded oxygen atoms of the α-phosphate or analogous α-phosphorus containing group under physiological conditions.

The nucleoside diphosphate or nucleoside diphosphonate prodrug or nucleoside analogue diphosphate or nucleoside analogue diphosphonate prodrug, or a prodrug comprising an analogous phosphorus-containing group, according to the first aspect of the invention is cell-penetrating and permits a slow intracellular release of an active compound, for example an antivirally or an antitumoral active compound. The prodrugs of the invention slowly release the corresponding nucleoside or nucleoside analogue monophosph(on)ates (NMPs), or the corresponding drug with the analogous phosphorus-containing group at the sugar component or sugar analogous component, which can intracellularly be converted into the corresponding triphosph(on)ates that serve as substrate for e.g. microbial/viral enzymes, for example, reverse transcriptase, in order to interfere with, for example, replication processes of pathogenic bacteria or viruses. In addition, the nucleoside diphosphate or nucleoside diphosphonate prodrug or nucleoside analogue diphosphate or nucleoside analogue diphosphonate prodrug, or the prodrug comprising an analogues phosphorus-containing group, according to the invention can itself, i.e., in the form of the prodrug, be an antivirally or antitumoral active compound. The moieties A and B, also referred to as masks, provide the prodrug of the invention with enough lipophilicity to allow the prodrug to enter the cell through the cell membrane. Adjustment of the necessary lipophilicity of the prodrug can be controlled by the structure of the moieties A and/or B. For example, by manipulating the size and/or length of a hydrocarbon residue. How the lipophilicity of a chemical group or molecule can be adjusted is known to a skilled person and can, if necessary, be determined by routine experimentation.

The compounds according to the invention can, for example, find advantageous use in medicinal products both as antiviral and as antitumoral agents. They are particularly suitable as drugs for the treatment of infections by viruses, in particular retroviruses such as HIV, influenza, hemorrhagic fever and hepatitis viruses.

The compounds according to the invention are generally present as salts under physiological conditions. In the following, a diphosphate or diphosphate analogue prodrug (Ia) is shown by way of example, where Cat⁺ means cation, for example ammonium, n-butylammonium, triethylammonium, a sodium or potassium cation. Nucl stands for nucleoside or nucleoside analogue.

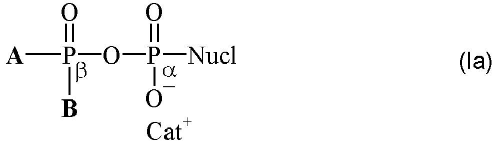

The term "Nucleoside diphosphate or nucleoside diphosphonate or a nucleoside analogue diphosphate or nucleoside analogue diphosphonate", also used in the form "nucleoside diphosph(on)ate or nucleoside analogue diphosph(on)ate", refers generally to a compound according to the following general structure (III) 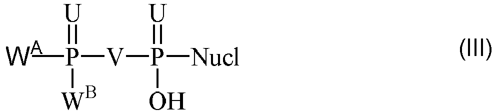
wherein U is, independently from each other, O, S, Se, or BH₃,
V is O, CH₂, NH, CHF, CHCI, CHBr, CF₂, CCl₂, CBr₂ or CFCI, and
W^{A} is H or HZ^{A} and W^{B} is H or HZ^{B} are, wherein Z^{A} and Z^{B} are, independent from each other, O, S, NH, CH₂, C=O (Carbonyl) or CR⁴R⁵, wherein R⁴, R⁵ are, for example, independently from each other, C₁₋₁₀ alkyl. The term thus not only encompasses nucleoside diphosphates or nucleoside analogue diphosphates, or nucleoside diphosphonates or nucleoside analogue diphosphonates in a narrower sense, i.e., compounds of formula III with U=O, V=O, W^{A}=HZ^{A}=OH, W^{B}=HZ^{B}=OH, and Nucl = nucleoside or nucleoside analogue in case of a nucleoside diphosphate or nucleoside diphosphate analogue, or compounds of formula III with U=O, V=O, W^{A}=H, W^{B}=HZ^{B}=OH (or, alternatively, W^{A}=HZ^{A}=OH, W^{B}=H), and Nucl = nucleoside or nucleoside analogue in case of a nucleoside diphosphonate or nucleoside analogue diphosphonate. The term "nucleoside analogue diphosphate or nucleoside analogue diphosphonate" ("nucleoside analogue diphosph(on)ate") also encompasses, for example, compounds wherein the nucleoside is a naturally occurring nucleoside and not a nucleoside analogue, but wherein the group containing the two phosphorus atoms is not a diphosphate or diphosphonate group, but a group being analogous to diphosph(on)ate group, i.e. group other than a diphosph(on)ate group but containing two phosphorus atoms and having a function essentially analogous to a diphosphate or diphosphonate group. Further, the term diphosphonate is also not to be construed as meaning that two phosphonate groups have to be or are present. Rather, the term relates to a compound containing a terminal phosphonate group, i.e. a phosphonate group or analogous group as the terminal phosphorus-containing group. The term "nucleoside analogue diphosph(on)ate" thus also refers to compounds not being diphosphates or diphosphonates in a strict sense, because they do not contain two phosphate or phosphonate groups, i.e. groups having a phosphorus atom bound to four oxygen atoms (in the case of a diphosphate) or bound to three oxygen atoms (in the case of a diphosphonate), but contain groups analogous to phosphate or phosphonate groups, e.g. phosphinate groups, instead of one or two phosphate groups. The term "nucleoside diphosph(on)ate or nucleoside analogue diphosph(on)ate" thus also relates to nucleosides having, covalently bound to their sugar component or the chemical group analogous to the sugar component, a group containing two phosphorus atoms being analogous to a diphosphate or diphosphonate group of a nucleoside diphosphate or diphosphonate.

Nucleoside diphosph(on)ates in a narrow sense are composed of a base component (nucleobase), a sugar component, e.g. a pentose like ribose or deoxyribose, and a diphosph(on)ate residue (diphosph(on)ate component) attached to an O-atom of the sugar component, e.g. an O-atom bound to the 5' atom of the pentose. The term "nucleoside analogue diphosph(on)ate" encompasses compounds containing a component analogous to a base component and/or a sugar component and/or a diphosph(on)ate component, i.e. also compounds comprising a naturally occurring nucleobase or a nucleobase analogue and a phosphate-sugar analogue. A "nucleoside analogue diphosph(on)ate" is a chemical compound, which is structurally and/or functionally similar to a nucleoside diphosph(on)ate such that an enzyme, e.g., a viral polymerase, having a nucleoside diphosphate naturally occurring in, for example, a human cell as a substrate would also use said compound as a substrate, analogous to the naturally occurring nucleoside diphosphate. The term "nucleoside analogue diphosphate" should not be construed as only relating to nucleoside analogue diphosphates in a narrow sense as defined above, and as excluding, for example, nucleoside diphosphonates or nucleoside analogue diphosphonates.

The terms "terminal phosphate group, phosphonate group or analogous phosphorus-containing group" or "terminal phosphate, phosphonate or analogous group" refer to any phosphate, phosphonate or analogous chemical group containing the terminal phosphorus atom, i.e. the phosphorus atom furthest from the point of attachment of the phosphorus-containing groups to the rest of the molecule. In a nucleoside diphosph(on)ate or nucleoside analogue diphosph(on)ate, the term relates to the β-phosphate (β-phosphoryl) group of the diphosphate group or the β-phosphonate group. The term "analogous phosphorus-containing group" relates to any chemical group, e.g. a phosphinate, phosphorothioate, phosphoroselenoate or boranophosphate group, being analogous to the terminal phosphate group or phosphonate group, i.e. the terminal group not being a phosphate group or phosphonate group but containing a phosphorus atom. The term "β-phosphorus atom" as used herein relates to the phosphorus atom of the terminal phosphorus containing group of the diphosph(on)ate component or the component being analogous thereto.

The term "nucleoside" refers to organic molecules consisting of a sugar residue (sugar component) linked to an organic base (base component), e.g. a heterocyclic organic base, in particular a nitrogen-containing heterocyclic organic base (nucleobase), which are linked via a glycosidic bond but may also be linked by a carbon-carbon bond (C-nucleoside). The sugar moiety is often a pentose, e.g. deoxyribose or ribose, but may also be another sugar, e.g. a C3-, C4- or C6-sugar. Frequently, but not exclusively, nucleobases are purines (R) or pyrimidines (Y). Examples of naturally occurring purines are guanine (G) and adenine (A), examples of naturally occurring pyrimidines are cytosine (C), thymine (T) and uracil (U). In particular, a nucleoside is therefore a compound of the following general formula (C): 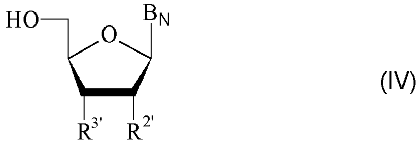 wherein B_{N} is a nitrogen-containing heterocyclic organic base, e.g. a nucleobase, and R^{2'} and R^{3'} are, independently from each other, H or OH. Phosphorylated nucleosides, for example nucleoside monophosphates (NMP), nucleoside diphosphates (NDP) and nucleoside triphosphates (NTP), are also referred to as nucleotides. The phosphate, diphosphate (pyrophosphate) or triphosphate group is generally linked to the O atom attached to the 5'-C atom of the sugar component of the nucleoside. However, the invention also encompasses compounds in which the phosphate group(s) is(are) bound to a 2'- or 3'-OH group. Additionally, it is possible that the pentose does not contain any heteroatoms in the ring (carbocyclic nucleosides) and/or that no substituents R²' or R³' are present and there is a double bond in the ring.

A "nucleoside analogue" (or "nucleoside analog") is to be understood here as an organic compound which is naturally not present in the human body but which is structurally similar to a nucleoside occurring naturally in the human body so that it can be used, for example, by the cell and/or viral enzymes essentially corresponding to the natural nucleoside, for example phosphorylated and incorporated into an RNA or DNA strand. A nucleoside analogue can itself be a nucleoside. However, for example, it may also be another compound having the above characteristics, for example a compound of a heterocyclic base and an acyclic residue and/or a residue which is not a sugar, or a compound of a carbocyclic compound and a heterocyclic base. In the case of carbocyclic nucleoside analogues, for example, the ring oxygen in the sugar component is replaced by a carbon (a methylene group or a substituted methylene group). In the sugar component, for example, a carbon atom may also be replaced by a heteroatom, for example the 3'-carbon atom by sulfur. Further, the 5' hydroxyl group of the sugar component may be replaced by another group, e.g., an amino group, or substituted. A nucleoside analogue may also be composed of a sugar like ribose or deoxyribose and a nucleobase analogue. Examples of nucleobase analogues are 5-bromouracil, 6-azathymine, 5-fluorouracil, 5-methylisocytosine and 6-fluor-3-hydroxy-2-pyrazincarboxamid (Favipiravir). Nucleoside analogues are either themselves nucleosides in the above sense or structurally and/or functionally analogous to nucleosides. Since nucleoside analogues do not necessarily have to contain a sugar or base component in the narrow sense, the terms "component analogous to the (naturally occurring nucleo)base" (base analogue) or "component analogous to the sugar component" (sugar analogue) may also be used here. If a sugar component or base component is mentioned here, the corresponding analogous components of nucleoside analogues are also encompassed, unless the context clearly indicates otherwise.

The term "non-bioreversible" or "intracellularly stable" in relation to the moieties A and B refer to a chemical group that is, under physiological conditions, i.e. under intracellular or in-vivo conditions, not or at least essentially not cleaved or cleaved off from the terminal phosph(on)ate or analogous phosphorus-containing group to which it is attached by an enzymatic reaction, in particular not by intracellular esterases, or by chemical hydrolysis. The term "physiological conditions" relates to the conditions normally occurring within a living cell, and refer, for example, to a temperature range of 20-40 °C, in particular 30-40 °C, preferably 36-40 °C, e.g. 37 °C, atmospheric pressure of 1, pH 6-8, in particular 7-7.5, and atmospheric oxygen concentration. If the term "hydrolytically stable" is used here in relation to the moieties, this means that the moieties are internally, i.e. intramolecularly, not or essentially not cleaved by hydrolysis under physiological conditions. This does not exclude a chemical cleavage as defined in the following. The term "chemically cleavable" in relation to the prodrugs of the invention relates to the fact that the prodrugs are cleavable by chemical means, e.g. hydrolysis, albeit slowly, between the α- and the β-phosphorus atom, thus releasing a nucleoside or nucleoside analogue monophosphate, for example. In this manner, the moieties A and B are slowly detached from the prodrug of the invention. The terms "chemically cleavable and non-bioreversible", "non-bioreversible but chemically cleavable", or "chemically cleavable, non-bioreversible and hydrolytically stable" may thus also be used in relation to the moieties A and B, but with the meaning, however, that the moieties A and B are not, under physiological conditions, cleaved themselves or cleaved off by cleaving the bond between the moieties A or B and the terminal phosphorus atom of the nucleoside (analogue) diphosph(on)ate, neither chemically nor enzymatically, but can nevertheless be cleaved off by chemical cleavage of the bond between the two phosphorus atoms of the two phosphorus atoms containing groups, e.g. phosphate groups. The term does not exclude that the moieties are also subject to an enzymatic cleavage as a result of a side reaction, but the cleavage is much slower than would be expected for a main enzymatic reaction. In particular, a "chemically cleavable, non-bioreversible and hydrolytically stable" moiety or an "intracellularly stable moiety" in relation to a prodrug of the invention is understood to be a moiety that is only slowly cleaved off from the prodrug under physiological conditions in a living cell, in particular human cell, such that the prodrug, i.e, the compound with the moieties A and B bound to it, has a high half-life in the cell, for example a half-life of >6 h, >8 h, >10 h, >24 h or >48 h, preferably >60, >72, >84, >96, >108 or >120 h.

If the abbreviation "Nucl" is used here, it encompasses both nucleosides and nucleoside analogues. The abbreviations "NMP", "NDP" and "NTP" encompass not only nucleoside monophosphates, nucleoside diphosphates and nucleoside triphosphates, but also corresponding analogues, i.e. nucleoside analogue monophosphates, nucleoside analogue diphosphates and nucleoside analogue triphosphates, or the corresponding phosphonates, unless explicitly stated otherwise.

For the purposes of the present invention, the indication of a range such as "1-10", for example, is to be understood as meaning that each intermediate value is also disclosed. In the case of an indication which can only affect integers, such as, for example, a number of C atoms, this means, of course, that only integers are disclosed. Any narrower range from a broader range is also meant to be disclosed by indicating the broader range, the narrower range also including ranges not comprising any of the boundary values of the broader range (e.g. a range of 2-5 from a range of 1-10).

The term "Cₙ-Cₘ" or "Cₙ₋ₘ", where n and m are each positive integers and m is greater than n, means a range indicating the number of carbon atoms of a compound or residue. The expression here expressly includes all integer intermediate values between the range boundaries n and m, in each case independently of one another. The expression "C₁₋₁₀" (n = 1, m = 10) therefore means, for example, a compound, group or residue having 1-10, i.e. 1, 2, 3, 4, 5, 6, 7, 8, 9 or 10 carbon atoms. "C₁₋₁₀" therefore also comprises, for example, "C₂₋₆", i.e. 2, 3, 4, 5 or 6 carbon atoms, or "C₁₋₄", i.e. 1, 2, 3 or 4 carbon atoms, or "C₄₋₉", i.e. 4, 5, 6, 7, 8 or 9 carbon atoms. Correspondingly, the term "C₁₋₂₀-alkyl" means, for example, an alkyl group having 1, 2, 3, 4, 5, 6, 7, 8, 9, 10, 11, 12, 13, 14, 15, 16, 17, 18, 19 or 20 carbon atoms and comprises all combinations of the values of n and m, which lie in the range from n = 1 to m = 20, for example "C₁₋₁₀ alkyl", i.e., an alkyl having 1, 2, 3, 4, 5, 6, 7, 8, 9 or 10 carbon atoms, or "C₅₋₇ alkyl", i.e. an alkyl having 5, 6 or 7 carbon atoms. Correspondingly, this also applies to terms such as "C₂₋₁₀ alkenyl", "C₄₋₂₀ alkenynyl", and the like.

The term "aliphatic residue" comprises cyclic or acyclic linear (straight chain) or branched, saturated or unsaturated carbon compound residues, other than aromatic residues. The term "heteroaliphatic residue" means aliphatic residues in whose carbon skeleton one or more C atoms are replaced by heteroatoms, for example oxygen, sulfur, nitrogen or phosphorus.

The term "alkyl" comprises saturated aliphatic (non-aromatic) groups including straight-chain (linear) alkyl groups (e.g. methyl, ethyl, propyl, butyl, pentyl, hexyl, heptyl and octyl), and branched alkyl groups (e.g. isopropyl, tert-butyl, isobutyl). The term also encompasses O, N, S or P alkyl groups (e.g., -O-methyl), i.e. alkyl groups which are bound to a compound via an oxygen, nitrogen, sulfur or phosphorus atom.

The term "alkenyl" comprises unsaturated aliphatic (non-aromatic) groups having at least one C-C double bond, including straight-chain and branched alkenyl groups. The term also includes O-, N-, S- or P-alkenyl groups (e.g., -O-propenyl), i.e. alkenyl groups bound to a compound via an oxygen, nitrogen, sulfur or phosphorus atom.

The term "alkynyl" (or "alkinyl") includes unsaturated aliphatic (non-aromatic) groups having at least one C-C triple bond, including straight-chain and branched alkynyl groups. The term also includes O, N, S or P alkynyl groups (e.g., -O-butinyl), i.e. alkynyl groups bound to a compound via an oxygen, nitrogen, sulfur or phosphorus atom.

The term "alkenynyl" (or "alkeninyl") includes unsaturated aliphatic (non-aromatic) groups having at least one C-C double bond and at least one C-C triple bond, including straight-chain and branched alkenynyl groups. The term also includes O-, N-, S-, or P-alkenynyl groups, i.e. alkenynyl groups bound to a compound via an oxygen, nitrogen, sulfur or phosphorus atom.

The term "cycloalkyl" includes compounds containing an alicyclic group, i.e. a ring-shaped saturated aliphatic (non-aromatic) group, e.g. cyclopropyl, cyclopentyl, cyclohexyl, cycloheptyl, cyclooctyl group. The term also includes O-, N-, S- or P-cycloalkyl groups, i.e. cycloalkyl groups bound to a compound via an oxygen, nitrogen, sulfur or phosphorus atom. The terms "cycloalkenyl", "cycloalkynyl" and "cycloalkenynyl" mean correspondingly ring-shaped aliphatic (non-aromatic) alkenyls, alkynyls or alkenynyls as defined above, the double and/or triple bond(s) being present within or outside the ring or ring system.

The term "heteroalkyl" refers to alkyl groups in which one or more carbon atoms of the hydrocarbon structure are replaced by other atoms (heteroatoms), e.g. oxygen, nitrogen, sulfur or phosphorus atoms. The term also includes O-, N-, S- or P-heteroalkyl groups, i.e. heteroalkyl groups which are bound to a compound via an oxygen, nitrogen, sulfur or phosphorus atom. The term "heteroalkyl" also includes cycloalkyls in which one or more carbon atoms of the hydrocarbon structure are replaced by other atoms, e.g. oxygen, nitrogen, sulfur or phosphorus atoms. The terms "heteroalkenyl", "heteroalkynyl", "heteroalkeninyl" mean corresponding alkenyls, alkynyls and alkeninyls, as well as cycloalkenyls, cycloalkynyls and cycloalkeninyls, in which one or more carbon atoms of the hydrocarbon skeleton are replaced by other atoms (heteroatoms), e.g. oxygen, nitrogen, sulfur or phosphorus atoms. For example, the term "C₁₋₂₀-heteroalkyl" means an alkyl group having 1, 2, 3, 4, 5, 6, 7, 8, 9, 10, 11, 12, 13, 14, 15, 16, 17, 18, 19 or 20 carbon atoms and at least one heteroatom. The same also applies to heteroalkenyls, heteroalkynyls and heteroalkenynyls.

By "aryl" are meant groups with aromaticity, including multi-membered aromatic single ring groups and multicyclic systems with at least one aromatic ring. Examples of aryl groups include benzene, phenyl and naphthalene. The term also includes O-, N-, S- or P-aryl groups, i.e. aryl groups bound to a compound via an oxygen, nitrogen, sulfur or phosphorus atom.

By "heteroaryl" are meant aryl groups having at least one heteroatom in the ring structure, i.e. in which one or more carbon atoms in the ring structure are replaced by other atoms (heteroatoms), e.g. b oxygen, nitrogen, sulfur or phosphorus atoms. Examples of heteroaryls are pyrrole, furan, thiophene, thiazole, isothiazole, imidazole, triazole, tetrazole, pyrazole, oxazole, pyridine, pyrazine, pyridazine and pyrimidine. The term also includes multicyclic, e.g. bicyclic and tricyclic, aryl groups, e.g. benzoxazole, benzodioxazole, benzothiazole, benzoimidazole, benzothiophene, methylenedioxyphenyl, quinoline, isoquinoline, indole, benzofuran, purine or benzofuran. The term also includes O-, N-, S- or P-heteroaryl groups, i.e. heteroaryl groups bound to a compound via an oxygen, nitrogen, sulfur or phosphorus atom.

By the term "halogen" is meant chlorine (CI), fluorine (F), bromine (Br) and iodine (I), in particular chlorine (CI) and fluorine (F).

The term "substituted" means that one or more substituents are present, which replace a hydrogen atom on one or more carbon atoms of the hydrocarbon structure or on one or more heteroatoms in the carbon skeleton. Examples of such substituents are oxo, hydroxyl, phosphate, cyano, azido and amino groups, but also e.g. halogens (e.g., F, Cl, Br), acyl, acyloxy, alkyl, cycloalkyl, heteroalkyl, heterocycloalkyl, aryl and heteroaryl groups. Instead of the term "substituent" also the terms "residue" or "moiety" may be used here.

A nucleoside diphosph(on)ate prodrug or nucleoside diphosph(on)ate analogue prodrug of the invention preferably has the general formula I 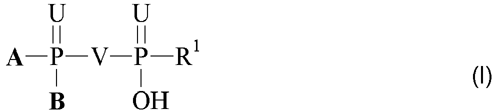 or a pharmaceutically acceptable salt thereof, wherein
A and B are both non-bioreversible and hydrolytically stable lipophilic moieties,
U is, independently from each other, O, S, Se, or BH₃, preferably O,
V is O, CH₂, NH, CHF, CHCI, CHBr, CF₂, CCl₂, CBr₂ or CFCI, preferably O,
and wherein R¹ is a nucleoside or nucleoside analogue.

Preferably, the moiety A of the nucleoside diphosph(on)ate prodrug or nucleoside diphosph(on)ate analogue prodrug has the formula (Ila) 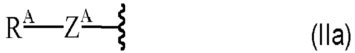 wherein,
Z^{A} is O, S, NH, CH₂, C=O (Carbonyl) or CR²R³, preferably O or CH₂, wherein R², R³ are, independently from each other, C₁₋₁₀ alkyl, and
R^{A} is a substituted or unsubstituted cyclic, acyclic, linear or branched aliphatic or heteroaliphatic residue, or a substituted or unsubstituted aromatic or heteroaromatic residue.

Further preferred, the moiety B of the nucleoside diphosph(on)ate prodrug or nucleoside diphosph(on)ate analogue prodrug has the formula (IIb) 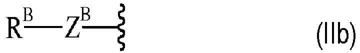 wherein,
Z^{B} is O, S, NH, CH₂, C=O (Carbonyl) or CR⁴R⁵, preferably O or CH₂, wherein R⁴, R⁵ are, independently from each other, C₁₋₁₀ alkyl, and
R^{B} is a substituted or unsubstituted cyclic, acyclic, linear or branched aliphatic or heteroaliphatic residue, or a substituted or unsubstituted aromatic or heteroaromatic residue.

The moieties A and B can be designed in a suitable manner such that the resulting prodrug can enter the cell via the cell membrane, and only slowly releases the corresponding nucleoside monophosphate, for example, intracellularly. The moieties A and B are designed in such a manner that a suitable lipophilicity is achieved. It is, for example, preferred that the moieties A and B themselves are not charged under physiological conditions, and thus do not contain one or more chemical groups that give rise to an electrical charge under physiological conditions.

The moieties A and B covalently attached to the terminal (beta) phosphorus atom, i.e. to the group containing the β-phosphorus atom of the nucleoside diphosph(on)ate or nucleoside analogue diphosph(on)ate prodrug, or pharmaceutically acceptable salt thereof, can be different or identical. In case of identical moieties terms like "symmetrically masked", "symmetrically modified" or "symmetrically protected" may also be used. In case the moieties A and B are different from each other, the term "non-symmetrically" may also be used in relation to the masking. Preferably, the moieties A and B at the terminal phosphorus atom are different from each other, e.g. different in length or size or the number and/or composition of their atoms. Different moieties within a single prodrug may be chosen depending on different functional properties of the moieties, e.g. as regards lipophilicity, charge neutralization capacity and/or reactivity, e.g., in terms of inhibitory activity.

In a preferred embodiment of the nucleoside diphosphate or nucleoside diphosphonate prodrug, or nucleoside analogue diphosphate or nucleoside analogue diphosphonate prodrug according to the invention, or a pharmaceutically acceptable salt thereof, the residues R^{A} and R^{B}
i. are, independently from each other, a substituted or unsubstituted cyclic, acyclic, linear or branched C₁₋₂₅ aliphatic or C₁₋₂₅ heteroaliphatic residue, or a substituted or unsubstituted C₅₋₂₅ aromatic or C₃₋₂₅ heteroaromatic residue, or
ii. are, independently from each other, selected from the group consisting of substituted or unsubstituted C₁₋₂₅ alkyl, substituted or unsubstituted C₂₋₂₅ alkenyl, substituted or unsubstituted C₂₋₂₅ alkynyl, substituted or unsubstituted C₄₋₂₅ alkenynyl, substituted or unsubstituted C₃₋₂₅ cycloalkyl, substituted or unsubstituted C₃₋₂₅ cycloalkenyl, substituted or unsubstituted C₅₋₂₅ cycloalkynyl, substituted or unsubstituted C₅₋₂₅ cycloalkenynyl, substituted or unsubstituted C₁₋₂₅ heteroalkyl, substituted or unsubstituted C₂₋₂₅ heteroalkenyl, substituted or unsubstituted C₂₋₂₅ heteroalkynyl, substituted or unsubstituted C₄₋₂₅ heteroalkenynyl, substituted or unsubstituted C₅₋₃₀ aryl, substituted or unsubstituted C₃₋₃₀ heteroaryl.

Further preferred, the residues R^{A} and R^{B}
i. are, independently from each other, a substituted or unsubstituted cyclic, acyclic, linear or branched C₁₋₂₀ aliphatic or C₁₋₂₀ heteroaliphatic residue, or a substituted or unsubstituted C₅₋₂₀ aromatic or C₃₋₂₀ heteroaromatic residue, or
ii. are, independently from each other, selected from the group consisting of substituted or unsubstituted C₁₋₂₀ alkyl, substituted or unsubstituted C₂₋₂₀ alkenyl, substituted or unsubstituted C₂₋₂₀ alkynyl, substituted or unsubstituted C₄₋₂₀ alkenynyl, substituted or unsubstituted C₃₋₂₀ cycloalkyl, substituted or unsubstituted C₃₋₂₀ cycloalkenyl, substituted or unsubstituted C₅₋₂₀ cycloalkynyl, substituted or unsubstituted C₅₋₂₀ cycloalkenynyl, substituted or unsubstituted C₁₋₂₀ heteroalkyl, substituted or unsubstituted C₂₋₂₀ heteroalkenyl, substituted or unsubstituted C₂₋₂₀ heteroalkynyl, substituted or unsubstituted C₄₋₂₀ heteroalkenynyl, substituted or unsubstituted C5-24 aryl, substituted or unsubstituted C₃₋₂₄ heteroaryl.

In a preferred embodiment of the prodrug of the invention at least one of the moieties A or B does not contain any oxygen atom not directly bound to the phosphorus atom of the terminal phosphate, phosphonate or analogous phosphorus-containing group, preferably both moieties A and B do not contain any oxygen atom not directly bound to the phosphorus atom of the terminal phosphate, phosphonate or analogous phosphorus-containing group.

In a further preferred embodiment of the prodrug of the invention moiety A contains less C-Atoms than moiety B, or moiety A contains less C-Atoms than moiety A.

In a further preferred embodiment of the prodrug of the invention moieties A and B do not comprise any cycloalkanes and/or aromatic rings.

In a still further preferred embodiment of the prodrug of the invention R^{A} and R^{B} are, independently from each other, C₁₋₂₅ alkyl, preferably C₁₋₂₃ alkyl, further preferred C₁₋₂₀ alkyl.

A nucleoside diphosphate or nucleoside diphosphonate prodrug, or a nucleoside diphosphate or nucleoside diphosphonate analogue prodrug according to the invention can, for example, be a compound having the following general structure (V) 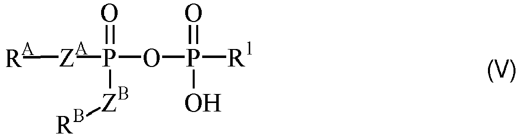 wherein R¹, R^{A}, R^{B}, Z^{A} and Z^{B} are as defined above, and wherein R^{A} and R^{B} are identical or different from each other. Preferably, R^{A} and R^{B} are, independently from each other, both alkyl, preferably C₁₋₂₅ alkyl, further preferred C₁₋₂₃ or C₁₋₂₀ alkyl. Preferably R^{A} and R^{B} are different from each other. R^{A} may be, for example, C₁₋₁₀ alkyl, preferably C₄₋₁₀ alkyl or C₄₋₈ alkyl, and R^{B} C₁₋₂₅ alkyl, preferably C₁₋₂₀ alkyl, further preferred C₄₋₂₀ or C₁₀₋₂₀ alkyl. Further preferred, Z^{A} is O or CH₂ and Z^{B} is O or CH₂, or Z^{A} is O and Z^{B} is O, or Z^{A} is CH₂ or OH and Z^{B} is CH₂, or Z^{A} is OH and Z^{B} is OH or CH₂. R¹ is nucleoside or nucleoside analogue.

A nucleoside diphosphate or nucleoside diphosphonate prodrug, or a nucleoside diphosphate or nucleoside diphosphonate analogue prodrug according to the invention may thus, for example, have one of the following general structures (Va to Vd): 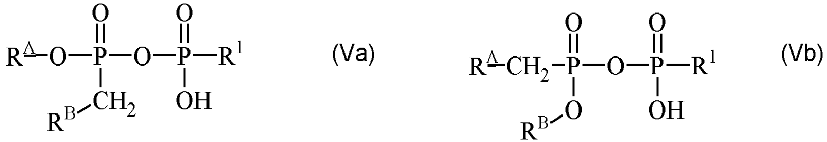 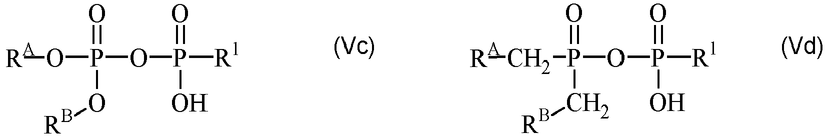 wherein R¹ is nucleoside or nucleoside analogue, and R^{A}, R^{B} are identical or different from each other, preferably different from each other, and wherein R^{A} and R^{B} are both alkyl, preferably, independently from each other, C₁₋₂₅ alkyl, further preferred C₁₋₂₃ or C₁₋₂₀ alkyl. Further preferred, R^{A} is C₁₋₁₀ alkyl, preferably C₄₋₁₀ alkyl or C₄₋₈ alkyl, and R^{B} is C₁₋₂₅ alkyl, preferably C₁₋₂₀ alkyl, further preferred C₄₋₂₀ or C₁₀₋₂₀ alkyl.

Preferably, the nucleoside or nucleoside analogue is selected from a group of nucleoside reverse transcriptase inhibitors (NRTI). Further preferred, the nucleoside or nucleoside analogue is selected from the group consisting of lamivudine (3TC), zidovudine (AZT), didanosine, stavudine (d4T), alovudine (fluorothymidine, FLT), FDU (FUdR, 3'-fluoro-2'-deoxyuridine), abacavir (ABC), amdoxovir (diaminopurine dioxolane, DAPD), apricitabin, elvucitabine, dioxolanthymine, fozivudine, fosalvudine, adefovir, tenofovir, cidovovir, entecavir (ETV) and emtricitabine (FTC).

Examples of a nucleoside diphosph(on)ate prodrug or nucleoside analogue diphosph(on)ate prodrug are listed in the Table 1 below (d4T = stavudine; AZT = zidovudine; FLT = alovudine, FDU = 3'-fluoro-2'-deoxyuridine; ABC = abacavir):

| No | Nucleoside Diphosph(on)ate prodrug | R¹ | Z^{A} | Z^{B} | R^{A} | R^{B} |
|---|---|---|---|---|---|---|
| 16 | 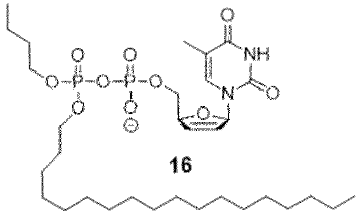 | d4T | O | O | C₄H₉ | C₁₈H₃₇ |
| 17 | 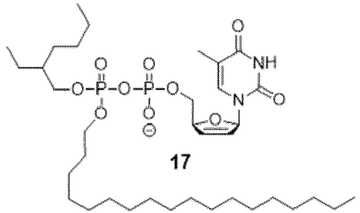 | d4T | O | O | C₈H₁₇ | C₁₈H₃₇ |
| 18 | 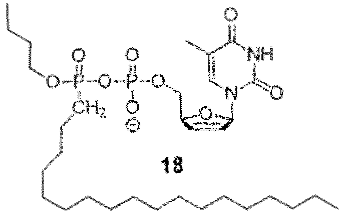 | d4T | O | CH₂ | C₄H₉ | C₁₇H₃₅ |
| 19 | 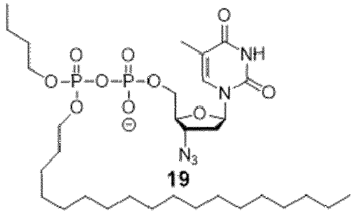 | AZT | O | O | C₄H₉ | C₁₈H₃₇ |
| 20 | 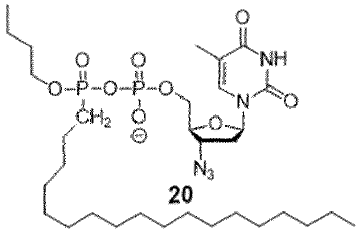 | AZT | O | CH₂ | C₄H₉ | C₁₇H₃₅ |
| 21 | 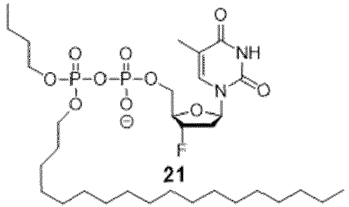 | FLT | O | O | C₄H₉ | C₁₈H₃₇ |
| 22 | 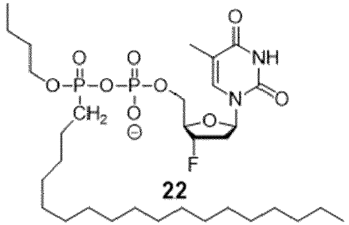 | FLT | O | CH₂ | C₄H₉ | C₁₇H₃₅ |
| 23 | 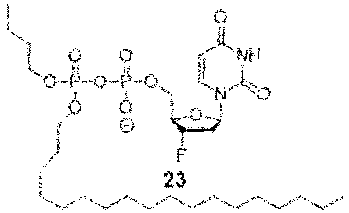 | FDU | O | O | C₄H₉ | C₁₈H₃₇ |
| 24 | 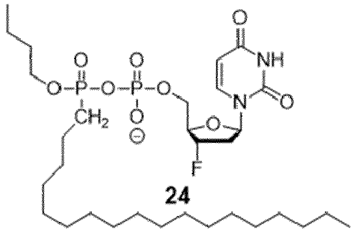 | FDU | O | CH₂ | C₄H₉ | C₁₇H₃₅ |
| 25 | 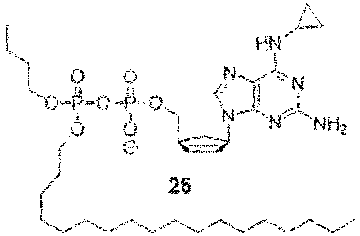 | ABC | O | O | C₄H₉ | C₁₈H₃₇ |
| 26 | 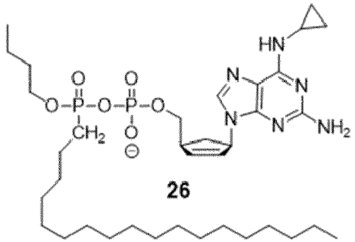 | ABC | O | CH₂ | C₄H₉ | C₁₇H₃₅ |

The invention also relates to a nucleoside diphosphate or nucleoside diphosphonate prodrug, or a nucleoside analogue diphosphate or nucleoside analogue diphosphonate prodrug according to the invention, or a pharmaceutically acceptable salt thereof, for use as a medicament. Preferably, the prodrugs or the salt thereof can be used as an antiviral medicament, more preferably as an antiretroviral medicament, for treating an HIV infection, hepatitis infection, influenza and/or hemorrhagic fever. The prodrugs of the invention are, however, also suitable for treating other diseases, for example cancer.

In a further aspect, the invention relates to a pharmaceutical composition or dosage form comprising a nucleoside diphosphate prodrug and/or a nucleoside diphosphonate prodrug, and/or a nucleoside analogue diphosphate prodrug and/or a nucleoside analogue diphosphonate prodrug according to one of the preceding claims, or a pharmaceutically acceptable salt thereof, and a pharmaceutically acceptable carrier.

Pharmaceutically acceptable carriers are known to the person skilled in the art and comprise one or more liquid, semi-solid or solid fillers, diluents or other substances suitable for administration to mammals, including humans.

For the purposes of the present invention, the term "carrier" refers to any organic or inorganic, natural or synthetic substance which can be combined with the active ingredient to simplify application. Examples of such carriers include, but are not limited to organic or inorganic solvents, starch, lactose, mannitol, methylcellulose, talc, gelatin, agar, calcium phosphate, magnesium stearate, animal and vegetable fats, higher molecular weight fatty acids, or higher molecular weight polymers.

The term "pharmaceutically acceptable" means any substantially non-toxic material for mammals, especially humans, which does not substantially affect the effectiveness of the biological activity of the active ingredient. Such materials may include pharmaceutically acceptable concentrations of salts, buffers, preservatives, or the like. Non-limiting examples of pharmaceutically acceptable carriers include magnesium carbonate, magnesium stearate, talc, sugar, lactose, ethanol, glycerol, water, buffer solutions. The pharmaceutical composition may also comprise adjuvants and/or diluents.

### Brief description of the figures:

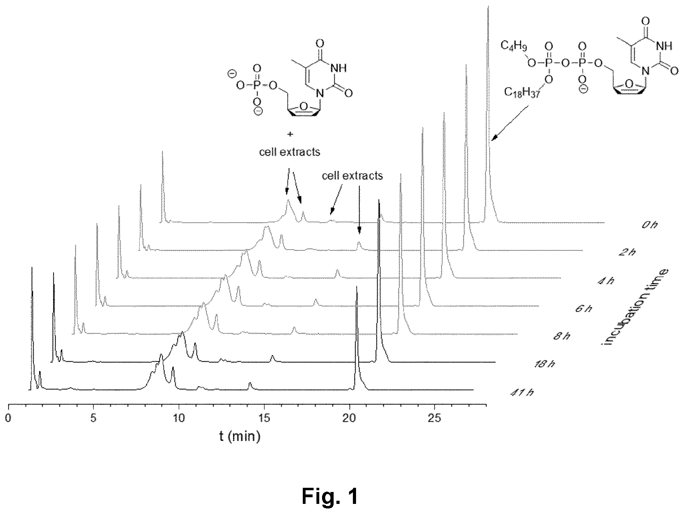

Fig. 1: Chromatogram showing the half live of an embodiment of a prodrug of the invention determined by RP-18 HPLC.

### Examples:

### A. Preparation of nucleoside diphosphate and of nucleoside diphosphonate

A simplified scheme of a synthesis route for a nucleoside diphosphate prodrug of the invention, wherein moiety A is O-C₄H₉ and moiety B is O-C₁₈H₃₇, and for a nucleoside diphosphonate, wherein moiety A is O-C₄H₉ and moiety B is C₁₈H₃₇, is shown in Scheme 1 below.

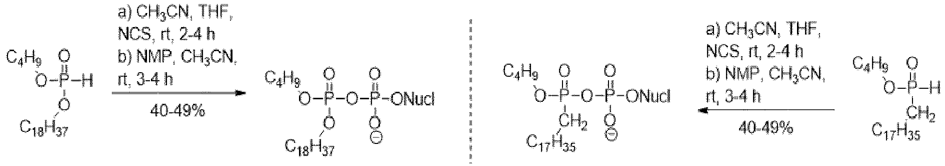

Nucleoside diphosph(on)ate prodrugs of the invention were prepared according to Scheme 1 above and tested.

### B. Stability test

Stability tests regarding a number of differently substituted β-phosphate and β-phosphonate nucleoside prodrugs were conducted. The chemical and biological stability was tested in different media (phosphate-buffered saline PBS (pH 7.3), pig liver esterase (PLE), CEM cell extracts) and rated by determining the half-live of these compounds. The products of the respective hydrolysis were detected by analytical RP-18 HPLC. Fig. 1 shows an example of a series of chromatograms for a nucleoside diphosphate prodrug, wherein moiety A is O-C₄H₉, moiety B is O-C₁₈H₃₇ and R₁ is stavudine (d4T), in CEM/0 cell extracts.

Results of the hydrolysis experiments in different media (PBS (pH = 7.3), PLE (pig liver esterase) in PBS, or CEM-T cell extracts) are shown in Table 2.

Table 2: Half-lives (in hours) of the β-phosph(on)ate prodrug compounds of the invention in phosphate buffer saline (PBS, pH 7.3), PLE (100 units/mL pig liver esterase in PBS) and CEM/0 cell extracts. n.d. = not determined.

| No: | Nucleoside Diphosph(on)ate prodrug | PBS | CEM T cell extracts | PLE |
|---|---|---|---|---|
| 16 | 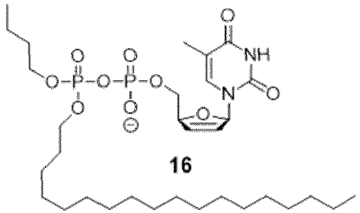 | >1000 | >18 | >150 |
| 17 | 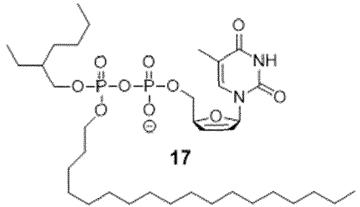 | >1000 | n.d. | n.d. |
| 18 | 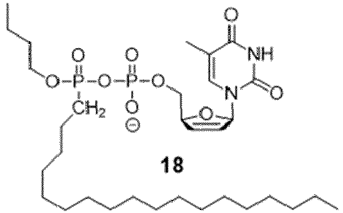 | >3000 | >40 | >150 |
| 19 | 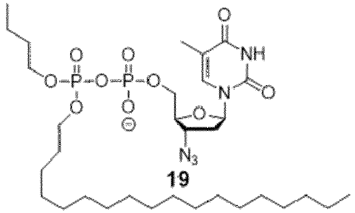 | 1028 | >8 | >150 |
| 20 | 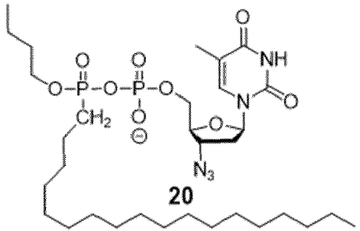 | >2000 | >18 | >150 |
| 21 | 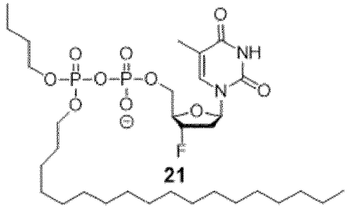 | >1000 | >10 | >150 |
| 22 | 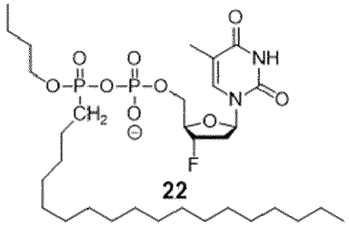 | >2000 | n.d. | n.d. |
| 23 | 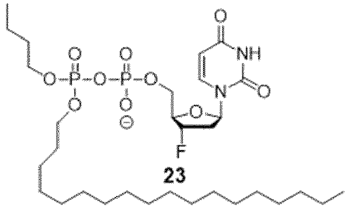 | 796 | n.d. | n.d. |
| 24 | 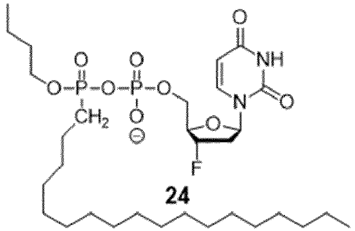 | >2000 | >20 | >150 |
| 25 | 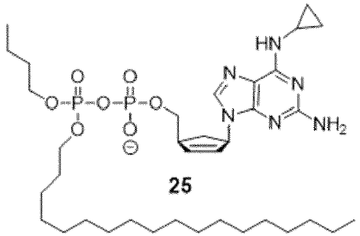 | 56 | 6.7 | 41 |
| 26 | 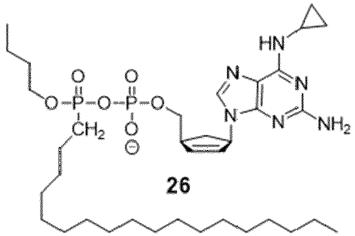 | >1000 | >10 | >150 |

### C. Antiviral activity and toxicity tests

All β-phosph(on)ate prodrug compounds were evaluated for their activity to inhibit the replication of HIV in HIV-1- and HIV-2-infected wild-type (CEM/0) cell cultures and in HIV-2-infected mutant thymidine kinase-deficient (CEM/TK⁻) cell cultures (see Table 3).

Table 3: Antiviral activity (EC₅₀ [µM]) of β-phosph(on)ate prodrug compounds of the invention compared to the respective nucleoside analogues in relation to HIV-1 and HIV-2. Antiviral activity was determined in CD4⁺ T-lymphocytes: 50% effective concentration (EC₅₀ [µM]). Values are the mean ±SD of n=2-3 independent experiments. CEM/TK⁻= thymidine kinase-deficient CD4⁺ T-cells: d4T = stavudine; AZT = zidovudine; FLT = alovudine, FDU = 3'-fluoro-2'-desoxyuridine; ABC = abacavir.

| No | Nucleoside Diphosph(on)ate prodrug | R¹ | HIV-1 (HE) | HIV-2 (ROD) | CEM/TK⁻ HIV-2 (ROD) |
|---|---|---|---|---|---|
| 16 | 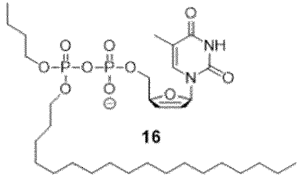 | d4T | 0.033 ±0.022 | 0.0044 ±0.0013 | 0.0011 ±0.0005 |
| 17 | 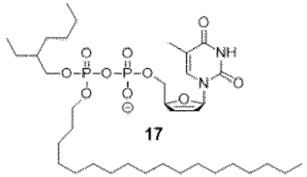 | d4T | 0.071 ±0.052 | 0.045 ±0.046 | 0.011 ±0.00071 |
| 18 | 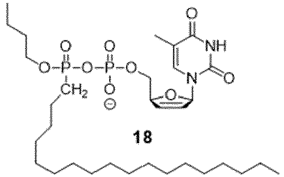 | d4T | 0.031 ±0.027 | 0.016 ±0.0083 | 0.0021 ±0.0034 |
| 19 | 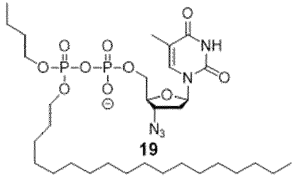 | AZT | 0.058 ±0.050 | 0.025 ±0.009 | 0.043 ±0.048 |
| 20 | 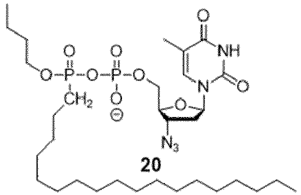 | AZT | 0.087 ±0.055 | 0.020 ±0.008 | 0.17 ±0.13 |
| 21 | 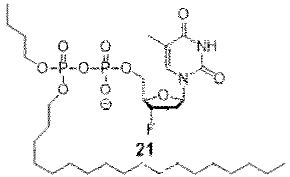 | FLT | 0.0067 ±0.0021 | 0.040 ±0.028 | 0.017 ±0.016 |
| 22 | 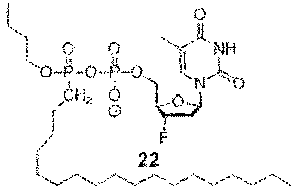 | FLT | 0.0063 ±0.0046 | 0.042 ±0.013 | 0.015 ±0.004 |
| 23 | 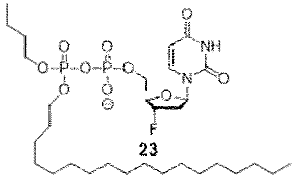 | FDU | 0.14 ±0.071 | 0.15 ±0.02 | 0.21 ±0.10 |
| 24 | 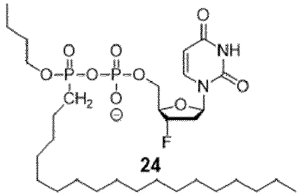 | FDU | 0.057 ±0.009 | 0.076 ±0.077 | 0.051 ±0.069 |
| 25 | 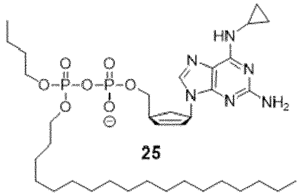 | ABC | 0.016 ±0.004 | 0.0087 ±0.0004 | 0.0031 ±0.0027 |
| 26 | 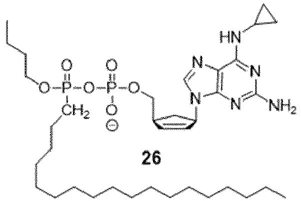 | ABC | 0.034 ±0.013 | 0.026 ±0.018 | <0.001 |
| | d4T | | 0.33 ±0.13 | 0.97 ±0.50 | >50 |
| | AZT | | 0.014 ± 0.0085 | 0.0034 ± 0.0021 | >100 |
| | FLT | | 0.0025 ±0.0029 | 0.0043 ±0.0017 | >100 ±0 |
| | FDU | | 0.21 ±0.02 | 0.15 ±0.017 | > 100 ±0 |
| | ABC | | 9.39 ±2.45 | 3.51 ±3.25 | 1.98 ±0.86 |

Moreover, the cytotoxicity of the antivirally active compounds was tested (see Table 4). Table 4 shows the cytotoxicity (CC₅₀ [µM]) of the β-phosph(on)ate compounds compared to the respective nucleoside analogues. In addition, a selectivity index (SI) was determined. The selectivity index is the ratio between cell toxicity and antiviral toxicity, the ratio between cell toxicity (see Table 4) and EC₅₀ CEM/TK⁻ (see right column of Table 3).

Table 4: Cytotoxicity (CC₅₀ [µM]) and selectivity index (SI) of tested prodrug compounds. Cytotoxicity: 50% cytostatic concentration or compound concentration required to inhibit CD4⁺ T-cell (CEM) proliferation by 50% (CC₅₀ [µM]); values are the mean ±SD of n=2-3 independent experiments. d4T = stavudine; AZT = zidovudine; FLT = alovudine, FDU = 3'-fluoro-2'-deoxyuridine; ABC = abacavir

| No | Nucleoside Diphosph(on)ate prodrug | R¹ | Toxicity | Selectivity index (SI) |
|---|---|---|---|---|
| 16 | 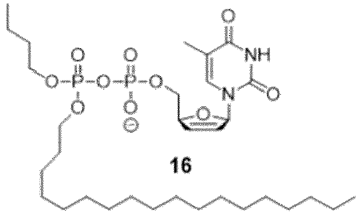 | d4T | 41.26 | 37509 |
| 17 | 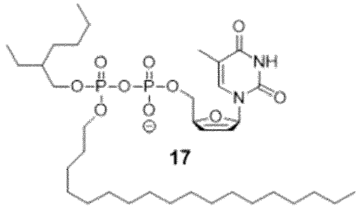 | d4T | 46.49 | 4226 |
| 18 | 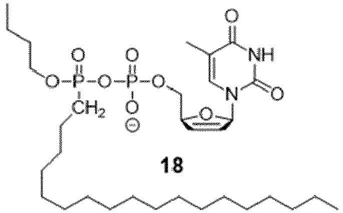 | d4T | 42.65 | 20309 |
| 19 | 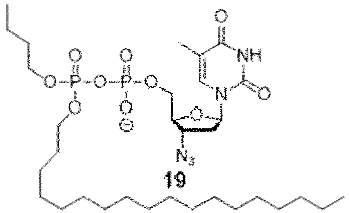 | AZT | 47.81 | 1112 |
| 20 | 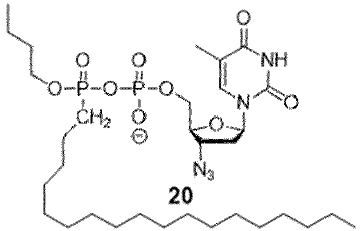 | AZT | 82.72 | 487 |
| 21 | 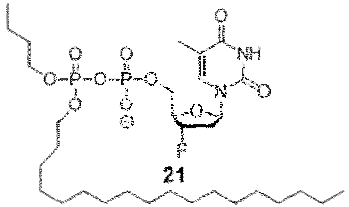 | FLT | 58.6 ±19.4 | 3447 |
| 22 | 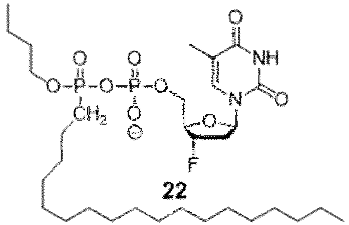 | FLT | 54.3 ±15.2 | 3620 |
| 23 | 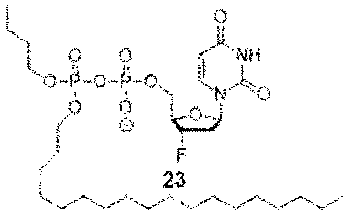 | FDU | 44.8 ±0 | 213 |
| 24 | 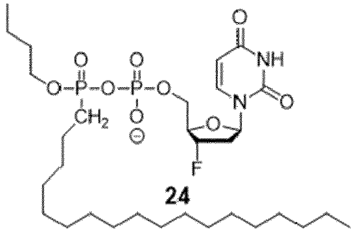 | FDU | 59.1 ±20.8 | 1159 |
| 25 | 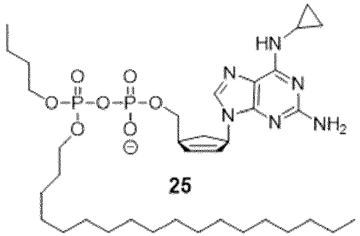 | ABC | 10.5 ±4.6 | 3387 |
| 26 | 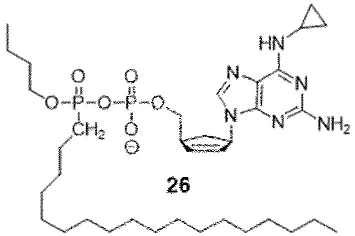 | ABC | >100 ±0 | >100000 |
| | d4T | | >50 | >1 |
| | AZT | | >100 | >1 |
| | FLT | | >100 ±0 | >1 |
| | FDU | | >100 ±0 | >1 |
| | ABC | | >100 | >50 |

All β-phosph(on)ate compounds bearing two different non-bioreversible alkyl residues were markedly antivirally active against HIV-2 in mutant thymidine-deficient (CEM/TK⁻) cells. For example, the antiviral activity of β-phosphate prodrug 16 (EC₅₀ = 0.0011 µM) in CEM/TK⁻ cells was improved by 45.400-fold as compared to the nucleoside analogue d4T (ECso > 50 µM). Further, the selectivity index of β-phosphate prodrug 16 (SI = 37.500) is much higher than that of the parent nucleoside d4T.

It was convincingly shown that the prodrugs of the invention provide have high potential in, for example, antiviral chemotherapies.

## Claims

1. A nucleoside diphosphate or nucleoside diphosphonate prodrug or a nucleoside analogue diphosphate or nucleoside analogue diphosphonate prodrug, or a pharmaceutically acceptable salt thereof, having two covalently bound lipophilic moieties A and B at the terminal phosphate group, phosphonate group or analogous phosphorus-containing group, wherein the lipophilic moieties A and B are both non-bioreversible.

2. A nucleoside diphosphate or nucleoside diphosphonate or a nucleoside analogue diphosphate or nucleoside analogue diphosphonate prodrug, having the general formula I 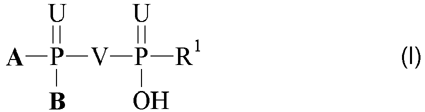 or a pharmaceutically acceptable salt thereof, wherein
A and B are both non-bioreversible lipophilic moieties,
U is, independently from each other, O, S, Se, or BH₃, preferably O,
V is O, CH₂, NH, CHF, CHCI, CHBr, CF₂, CCl₂, CBr₂ or CFCI, preferably O,
and wherein
R¹ is a nucleoside or nucleoside analogue.

3. A nucleoside diphosphate or nucleoside diphosphonate prodrug, or a nucleoside analogue diphosphate or nucleoside analogue diphosphonate prodrug, or a pharmaceutically acceptable salt thereof, according to claim 1 or 2, wherein
i) A is a residue according to formula Ila 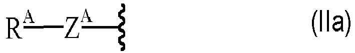 wherein,
Z^{A} is O, S, NH, CH₂, C=O (Carbonyl) or CR²R³, preferably O or CH₂, wherein R², R³ are, independently from each other, C₁₋₁₀ alkyl,
R^{A} is a substituted or unsubstituted cyclic, acyclic, linear or branched aliphatic or heteroaliphatic residue, or a substituted or unsubstituted aromatic or heteroaromatic residue,
and wherein
ii) B is a residue according to formula IIb 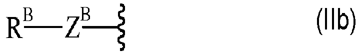 wherein,
Z^{B} is O, S, NH, CH₂, C=O (Carbonyl) or CR⁴R⁵, preferably O or CH₂, wherein R⁴, R⁵ are, independently from each other, C₁₋₁₀ alkyl, and
R^{B} is a substituted or unsubstituted cyclic, acyclic, linear or branched aliphatic or heteroaliphatic residue, or a substituted or unsubstituted aromatic or heteroaromatic residue.

4. A nucleoside diphosphate or nucleoside diphosphonate prodrug, or a nucleoside analogue diphosphate or nucleoside analogue diphosphonate prodrug according to one of claims 1 to 3, or a pharmaceutically acceptable salt thereof, wherein the lipophilic moieties A and B are different from each other.

5. A nucleoside diphosphate or nucleoside diphosphonate prodrug, or a nucleoside analogue diphosphate or nucleoside analogue diphosphonate prodrug according to one of claims 2 to 4, or a pharmaceutically acceptable salt thereof, wherein R^{A} and R^{B} i. are, independently from each other, a substituted or unsubstituted cyclic, acyclic, linear or branched C₁₋₂₅ aliphatic or C₁₋₂₅ heteroaliphatic residue, or a substituted or unsubstituted C₅₋₂₅ aromatic or C₃₋₂₅ heteroaromatic residue, or
ii. are, independently from each other, selected from the group consisting of substituted or unsubstituted C₁₋₂₅ alkyl, substituted or unsubstituted C₂₋₂₅ alkenyl, substituted or unsubstituted C₂₋₂₅ alkynyl, substituted or unsubstituted C₄₋₂₅ alkenynyl, substituted or unsubstituted C₃₋₂₅ cycloalkyl, substituted or unsubstituted C₃₋₂₅ cycloalkenyl, substituted or unsubstituted C₅₋₂₅ cycloalkynyl, substituted or unsubstituted C₅₋₂₅ cycloalkenynyl, substituted or unsubstituted C₁₋₂₅ heteroalkyl, substituted or unsubstituted C₂₋₂₅ heteroalkenyl, substituted or unsubstituted C₂₋₂₅ heteroalkynyl, substituted or unsubstituted C₄₋₂₅ heteroalkenynyl, substituted or unsubstituted C₅₋₃₀ aryl, substituted or unsubstituted C₃₋₃₀ heteroaryl.

6. A nucleoside diphosphate or nucleoside diphosphonate prodrug, or a nucleoside analogue diphosphate or nucleoside analogue diphosphonate prodrug according to claim 5, or a pharmaceutically acceptable salt thereof, wherein R^{A} and R^{B}
i. are, independently from each other, a substituted or unsubstituted cyclic, acyclic, linear or branched C₁₋₂₀ aliphatic or C₁₋₂₀ heteroaliphatic residue, or a substituted or unsubstituted C₅₋₂₀ aromatic or C₃₋₂₀ heteroaromatic residue, or
ii. are, independently from each other, selected from the group consisting of substituted or unsubstituted C₁₋₂₀ alkyl, substituted or unsubstituted C₂₋₂₀ alkenyl, substituted or unsubstituted C₂₋₂₀ alkynyl, substituted or unsubstituted C₄₋₂₀ alkenynyl, substituted or unsubstituted C₃₋₂₀ cycloalkyl, substituted or unsubstituted C₃₋₂₀ cycloalkenyl, substituted or unsubstituted C₅₋₂₀ cycloalkynyl, substituted or unsubstituted C₅₋₂₀ cycloalkenynyl, substituted or unsubstituted C₁₋₂₀ heteroalkyl, substituted or unsubstituted C₂₋₂₀ heteroalkenyl, substituted or unsubstituted C₂₋₂₀ heteroalkynyl, substituted or unsubstituted C₄₋₂₀ heteroalkenynyl, substituted or unsubstituted C5-24 aryl, substituted or unsubstituted C₃₋₂₄ heteroaryl.

7. A nucleoside diphosphate or nucleoside diphosphonate prodrug, or a nucleoside analogue diphosphate and/or nucleoside analogue diphosphonate prodrug according to one of the preceding claims, or a pharmaceutically acceptable salt thereof, wherein at least one of the moieties A or B does not contain any oxygen atom not directly bound to the phosphorus atom of the terminal phosphate, phosphonate or analogous phosphorus-containing group, preferably both moieties A and B do not contain any oxygen atom not directly bound to the phosphorus atom of the terminal phosphate, phosphonate or analogous phosphorus-containing group.

8. A nucleoside diphosphate or nucleoside diphosphonate prodrug, or a nucleoside analogue diphosphate or nucleoside analogue diphosphonate prodrug according to one of the preceding claims, or a pharmaceutically acceptable salt thereof, wherein moiety A contains less C-Atoms than moiety B, or wherein moiety A contains less C-Atoms than moiety A.

9. A nucleoside diphosphate or nucleoside diphosphonate prodrug, or a nucleoside analogue diphosphate or nucleoside analogue diphosphonate prodrug according to one of the preceding claims, or a pharmaceutically acceptable salt thereof, wherein moieties A and B do not comprise any cycloalkanes and/or aromatic rings.

10. A nucleoside diphosphate or nucleoside diphosphonate prodrug, or a nucleoside analogue diphosphate or nucleoside analogue diphosphonate prodrug according to one of claims 2 to 9, or a pharmaceutically acceptable salt thereof, wherein R^{A} and R^{B} are, independently from each other, C₁₋₂₅ alkyl, preferably C₁₋₂₃ alkyl, further preferred C₁₋₂₀ alkyl.

11. A nucleoside diphosphate or nucleoside diphosphonate prodrug, or a nucleoside analogue diphosphate or nucleoside analogue diphosphonate prodrug according to one of the preceding claims, or a pharmaceutically acceptable salt thereof, wherein the nucleoside or nucleoside analogue is selected from a group consisting of nucleoside reverse transcriptase inhibitors (NRTI).

12. A nucleoside diphosphate or nucleoside diphosphonate prodrug, or a nucleoside analogue diphosphate or nucleoside analogue diphosphonate prodrug according to one of the preceding claims, or a pharmaceutically acceptable salt thereof, for use as a medicament.

13. A nucleoside diphosphate or nucleoside diphosphonate prodrug, or a nucleoside analogue diphosphate or nucleoside analogue diphosphonate prodrug according to one the preceding claims, or a pharmaceutically acceptable salt thereof, for use as an antiviral medicament, preferably as an antiretroviral medicament, for treating an HIV infection, hepatitis infection, influenza or hemorrhagic fever.

14. Pharmaceutical composition or dosage form comprising a nucleoside diphosphate prodrug and/or a nucleoside diphosphonate prodrug, and/or a nucleoside analogue diphosphate prodrug and/or a nucleoside analogue diphosphonate prodrug according to one of the preceding claims, or a pharmaceutically acceptable salt thereof, and a pharmaceutically acceptable carrier.
